Europäisches Patentamt

European Patent Office

Office européen des brevets

(11) Numéro de publication : **0 440 555 A1**

(12) # DEMANDE DE BREVET EUROPEEN

(21) Numéro de dépôt : 91400227.4

(22) Date de dépôt : 31.01.91

(51) Int. Cl.⁵ : **B01J 27/18, C07C 29/124, C01B 25/26**

(30) Priorité : 02.02.90 FR 9001264

(43) Date de publication de la demande :
07.08.91 Bulletin 91/32

(84) Etats contractants désignés :
AT BE CH DE DK ES FR GB GR IT LI LU NL SE

(71) Demandeur : RHONE-POULENC CHIMIE
25, quai Paul Doumer
F-92408 Courbevoie Cédex (FR)

(72) Inventeur : Doussain, Claude
2, rue Louis Girardet
F-69190 Saint-fons (FR)

Inventeur : Gilbert, Laurent
72, rue Vauban
F-69006 Lyon (FR)
Inventeur : Gubelmann, Michel
39, Boulevard des Belges
F-69006 Lyon (FR)
Inventeur : Pernot, Hélène
Née Lauron, 141, Quai de Valmy
F-75010 Paris (FR)
Inventeur : Popa, Jean-Michel
2, rue Roger Breton
F-93700 Drancy (FR)
Inventeur : Tirel, Philippe-Jean
Rue des Chanturières
F-69360 Communay (FR)

(74) Mandataire : Ricalens, François et al
RHONE-POULENC CHIMIE Service Brevets
Chimie 25, quai Paul Doumer
F-92408 Courbevoie (FR)

(54) **Composé chimique contenant des alcalins ou des alcalino-terreux, catalyseur en contenant et procédé de transformation et notamment de solvolyse utilisant ledit catalyseur.**

(57)    L'invention vise des composés chimiques et leurs utilisations.
Ces composés chimiques répondent à la formule suivante sur sec :

$$(EO_4M), (Imp)p \quad (I)$$

avec E choisi dans le groupe des éléments de la colonne VB à l'exception de l'azote, de préférence le phosphore,
- où M est un métal ou un mélange de métaux.
Imp correspond à un composé d'imprégnation basique constitué de métal alcalino-terreux Alt, de préférence de métal alcalin Alc et leurs mélanges associés à un contreanion pour assurer la neutralité électrique.
Le coefficient p est compris entre $10^{-2}$ et 1/3.
Application à la catalyse de la solvolyse des halogénures d'alcoyle.

EP 0 440 555 A1

FIG.1a

# COMPOSE CHIMIQUE CONTENANT DES ALCALINS OU DES ALCALINO-TERREUX, CATALYSEUR EN CONTENANT ET PROCEDE DE TRANSFORMATION ET NOTAMMENT DE SOLVOLYSE UTILISANT LEDIT CATALYSEUR

La présente invention concerne un procédé de transformation et notamment de solvolyse en phase gazeuse d'halogénure organique par exemple pour préparation du trifluoroéthanol par hydrolyse, en phase gazeuse, du chloro-1 trifluoro-2,2,2 éthane.

La présente invention concerne aussi plus généralement de nouveaux composés chimiques utilisables notamment comme catalyseurs de solvolyse d'un halogénure d'alcoyle, surtout lorsque le carbone portant ledit halogène est au voisinage de groupement(s) attracteur(s), comme par exemple le groupement trifluorométhyle.

Le problème du passage du chlorure de trifluoro-2,2,2 éthyle à l'alcool correspondant, par une solvolyse où l'eau est le solvant, constitue un excellent exemple des difficultés rencontrées et de la solution apportée par la présente invention.

En d'autres termes, le chlorure de trifluoro-2,2,2 éthyle constitue un paradigme des halogénures d'alcoyle dont la solvolyse est d'ordinaire difficile et plus aisée selon la présente invention.

Ainsi le trifluoro-2,2,2 éthanol (TFE) est un alcool trifluoré possédant une très bonne stabilité thermique, ce qui rend apte à un certain nombre d'applications, notamment dans la synthèse d'anesthésiques fluorés, en pharmacologie en général et comme solvant.

On a décrit la préparation de cet alcool soit par hydrogénation de l'acide trifluoroacétique ou de ses esters, soit par hydrolyse de l'acétate de trifluoro-2,2,2 éthyle, en phase liquide dans un solvant comportant des groupes hydroxylés.

Ces divers procédés de préparation du TFE ne sont pas entièrement satisfaisants sur le plan industriel, de sorte qu'il a été engagé des recherches pour savoir s'il ne serait pas possible de réaliser l'hydrolyse directe, en phase gazeuse, du chloro-1 trifluoro-2,2,2 éthane. La demande Française non publiée déposée sous le numéro 88 10813 par la demanderesse apporte un progrès important, mais la sélectivité de la réaction et son rendement laissent à désirer.

C'est pourquoi un des buts de la présente invention est de fournir des catalyseurs qui introduisent une meilleure sélectivité et de bons rendements dans les procédés de solvolyse ci-dessus.

Un autre but de la présente invention est de fournir des composés chimiques qui utilisés en tant que catalyseur, conduisent à une bonne sélectivité et à de bons rendements lors d'une transformation telle que la solvolyse, par exemple l'hydrolyse, d'halogénure(s) d'alcoyle.

Un autre but de la présente invention est de fournir un procédé de fabrication d'un tel composé chimique.

Un autre but de la présente invention est un procédé de transformation telle que la solvolyse, par exemple l'hydrolyse, d'halogénure(s) d'alcoyle, utilisant les catalyseurs ci-dessus.

Ces buts et d'autres qui apparaîtront par la suite sont atteints au moyen de composés chimiques qui répondent à la formule suivante :

$$(EO_4M), (Imp)p \quad (I)$$

avec E choisi dans le groupe des éléments de la colonne VB à l'exception de l'azote (cf. définie dans le tableau de la Classification périodique des éléments publiée dans le " Bull. Soc. Chim. 1966 N°1) de préférence le phosphore,

- où M est un métal ou un mélange de métaux tel que :

$$M = \alpha\, M_1{}^+ + \beta\, M_2{}^{++} + \gamma\, M_3{}^{3+} + \delta M_4{}^{4+} \text{ avec la relation}$$

$$\alpha + 2\beta + 3\gamma + 4\delta = 3$$

$M_1$ est choisi dans le groupe des éléments de la colonne I A et leurs mélanges, de préférence les métaux alcalins (noté par la symbole Alc).Avantageusement Alc représente le potassium, le rubidium, le cesium et leurs mélanges, plus préférentiellement le potassium, le cesium et leurs mélanges.

$M_2$ est choisi parmi les éléments de transition divalents (voire le zinc et le cadmium,) les alcalino-terreux (Alt) et leurs mélanges. Avantageusement $M_2$ est composé majoritairement, voire (quasi) totalement d'alcalino-terreux.

Les Alt préférés sont le calcium, le strontium et le baryum, leurs mélanges et les mélanges les contenant.

$M_3$ est choisi parmi les éléments de transition trivalents, les éléments de la colonne III B, les éléments à sous-couche électronique f et assimilés, et leurs mélanges. Avantageusement $M_3$ est composé majoritairement, voire (quasi) totalement, d'éléments à sous-couche f et assimilés.

Parmi les métaux à sous-couche f et assimilés, on préfère les terres rares (yttrium, lanthane et lanthanides et surtout le lanthane), leurs mélanges et les mélanges les contenant.

$M_4$ est choisi parmi les terres rares tetravalentes, le titane, le hafnium et l'étain. $M_4$ peut également être germanium, voire silicium.

α     est un coefficient compris entre 0 et 3 avantageusement supérieur à 0,01 et au plus à 1/2, de préférence compris entre 0,05 et 0,2.

β     est un coefficient compris entre 0 et 3/2, de préférence entre 0 et 1/3 ou bien 1 ± 0,1.

γ     est un coefficient compris entre 0 et 1, avantageusement au moins égal à 1/3 de préférence à 1/2.

delta     est un coefficient compris entre 0 et 3/4, avantageusement entre 0 et 1/3, de préférence entre 0 et 1/6.

En général M est constitué d'au plus trois éléments et ce pour des raison de commodité ; pour les mêmes raisons il peut être économiquement intéressant d'utiliser des mélanges commerciaux de terres rares sous quelque forme qu'ils soient dès lors qu'ils conduisent aisément au composé selon l'invention ; ainsi bien que cela ne soit guère critique, il est fréquent que gamma soit peu différent de un (0,9 plus ou moins 0,1) et que M ne comporte qu'un métal aux impuretés près ;

Imp     correspond à un composé d'imprégnation basique constitué de métal choisi parmi les alcalins et alcalino-terreux, de préférence les métaux alcalins, et leurs mélanges associés à un ou plusieurs contre- anions pour assurer la neutralité électrique. Les contre anions n'ont que peu ou pas d'influence sur la catalyse.

Imp     est avantageusement différent de $MEO_4$, notamment lorsque l'imprégnant est un alcalino-terreux. L'imprégnant est qualifié de basique en raison du fait qu'il est dans la majorité des cas accepteur de proton, en tout cas après conditionnement à une température voisine de celle de la réaction de solvolyse.

Le(s) contre-anion(s) initial(aux), c'est à dire avant traitement thermique, est choisi de préférence parmi les halogénures de préférence le fluorure et leurs mélanges ; les ions OH⁻ et mélanges le contenant, de préférence majoritairement ; les dérivés et mélanges contenant des membres de la famille $EO_4$— tels que les phosphates et hydrogénophosphates et ceux qui le deviennent après pyrolyse tel que par exemple phospho- ou phosphinate.

En outre le(s) contre-anion(s) initial(aux) associé(s) peut avantageusement être choisi parmi les anions volatils, ou décomposables, tels que carbonate(s), nitrate, sulfate(s,), carboxylates, sulfonates ; ces anions sont en général décomposables dans les conditions de la solvolyse ou de la formation d'insaturé(s). Les anions qui en dérivent (par exemple les anions oxygénés tel que O⁻,OH⁻ ou ceux qui, tels les halogènures, appartenant à la phase réactionnnelle se substituent aux anions initiaux ou aux oxygénés) dans ces conditions, ou qui restent inchangés donnent des composés selon l'invention qui ont un bon pouvoir catalytique. Les anions peuvent être d'une seule espèce ou être constitués d'un mélange des espèces précitées ; pour des raisons de simplicité on préfère n'avoir qu'une seule espèce ou qu'une seule famille d'espèces.

Les composés selon la présente invention présente, notamment après conditionnement à la température de réaction, la propriété d'être de bon catalyseur de transformation des substrats selon la présente invention.

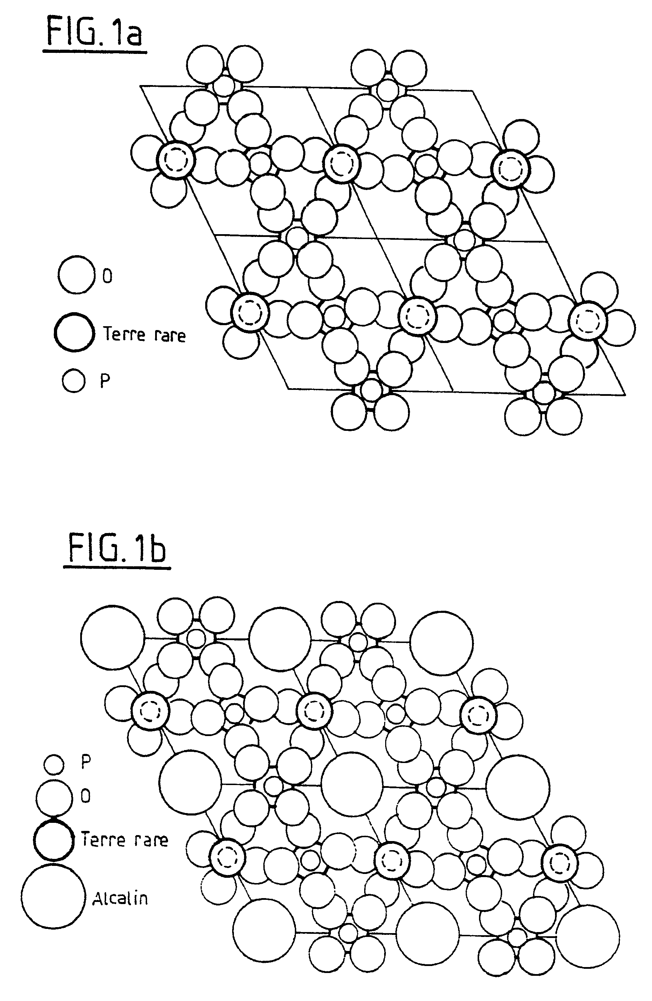

Pour les réactions de solvolyse notamment lorsque γ est ≧ à 1/2, de préférence à 2/3 les catalyseurs les meilleurs sont ceux dont la matrice ($MEO_4$) est de structure hexagonale ou monoclinique, de préférence ceux dont la matrice présente, au moins à basse température, une structure hexagonale (c'est-à-dire avec un axe de symétrie C6 parallèle aux canaux zéolithiques, le système de canaux étant unidimensionnel et non interconnecté, bien entendu compte non tenu des défauts structuraux) comme par exemple celle du type dessiné à la figure 1a. Plus préférentiellement il a été montré que les meilleurs catalyseurs était ceux où l'imprégnation est réalisée sur matrice au moins partiellement à structure de type hexagonal.

Enfin il est également préférable que par chauffage la structure hexagonale de la matrice soit transformable en structure Monoclinique et que après imprégnation le composé selon l'invention soit soumis à des conditions qui assure la transformation hexagonale-monoclinique.

Avantageusement les structures (hexagonales, monocliniques ou autres) des composés selon l'invention possèdent au moins en surface des pores, de préférence des canaux, dont la taille (diamètre) est comprise entre entre 0,2 et 0,5 nm de préférence entre 3 et 4 angstroms (0,3 et 0,4 nm).

Sans que cette explication soit limitative, il semblerait que le bon pouvoir catalytique observé pour les structures hexagonales fût corrélé avec le remplissage au moins en surface, lors de l'imprégnation, des canaux de la structure hexagonale par l'alcalin (comme indiqué dans la figure lb) ou l'alcalino-terreux provenant de l'impregnant et ce bien que, dans le cas où la structure finale était monoclinique, il ne restât aucune trace de la structure hexagonale qui fût décelable avec les moyens d'investigation usuels.

Il peut d'ailleurs être mentionné qu'il est parfois vain et très difficile de faire le départ après usage et/ou calcination, entre ce qui est matrice et ce qui est imprégnant. Dans les cas où après usage et/ou calcination, il est très difficile de faire le départ entre ce qui est matrice et ce qui est imprégnant, pour déterminer si un composé rentre dans le cadre de la présente invention on considerera le composé ($EO_4M$), (Imp)p (I) comme un tout chimique sans chercher à distinguer ($EO_4M$) de (Imp)p.

On peut incidemment signaler que si les anions, directs ou derivés, ne jouent qu'un rôle insignifiant dans la catalyse, ils peuvent avoir une importance sémiotique très grande car il sont le signe de l'existence d'"Imp" voire le moyen de déterminer "p", lorsque les anions utilisés sont distinguables des anions de la famille de la

matrice ($MEO_4$). A titre indicatif, dans le cas contraire, si la matrice n'est pas composée exclusivement de métaux alcalins, et si l'impregnant n'est exclusivement constitué d'alcalino-terreux, il est possible de se reporter à la contrainte $\alpha + p$ somme qui est en général inférieure ou égale à 3,3 et supérieur à $10^{-2}$, avantageusement comprise entre 0,05 et 1, de préférence entre 0,05 et 1/2. Pour les autres cas on peut utiliser tout indice, y compris le pouvoir catalytique.

Enfin parmi les caractéristiques notables des composés utilisables comme catalyseur il convient de signaler qu'il est avantageux que la concentration superficielle en alcalin soit comprise entre 1/4 et 8, de préférence entre 1 et 5 atome par $nm^2$ (concentration par unité de surface, l'épaisseur n'étant pas définie ; toutefois, on peut estimer que les alcalins de l'imprégnant ne devraient pas migrer au-delà de 10 à 20 nm de la surface et donc que l'épaisseur ne devrait pas dépasser sensiblement 10 à 20 nm ; pour les alcalino-terreux, on divisera la valeur des fourchettes par 2).

La sélectivité de la réaction est également dépendante de l'alcalin ou de l'alcalino-terreux. Plus la période à laquelle il appartient est de rang élevé, plus la réaction de solvolyse est favorisée ; en revanche plus le rang est faible, plus la réaction de déshydrohalogénation est favorisée (la troisième période, celle du sodium qui peut aisément être utilisée pour les deux réactions pourrait être considérée comme une frontière pratique) .

D'une manière générale les composés selon la présente invention qui donnent des sélectivités médiocres en solvolyse donne souvent des résultats de qualité inverse en déshydrohalogénation. En d'autres termes tout se passe comme si les substrats avaient le choix entre les deux voies principales possibles (solvolyse ou déshydrohalogénation) et que en choisissant les catalyseurs (selon les lignes directrices ci-dessus) on pouvait privilégier l'une ou l'autre des réactions.

Le coefficient p représente le rapport entre l'imprégnant exprimé en équivalent gramme et l'imprégné ($EO_4M$) exprimé en mole.

Le coefficient p est compris entre $10^{-2}$ et 1/3 avantageusement entre 0,05 et 1/4 de préférence entre $1/2.10^{-1}$ et 1/5.

En outre $\alpha + p$ est en général au plus égal à 3,3 et supérieur à $10^{-2}$, avantageusement entre 0,05 et 1, de préférence entre 0,05 et 1/2.

Pour ce qui est des Alc et Alt, les préférences sont les mêmes que celles exprimées précédemment.

Ces composés peuvent être fabriqués par imprégnation d'un composé de formule $EO_4M$, où M a été définit antérieurement, avec une solution ou une suspension d'Imp dans un solvant volatil, de préférence l'eau. L'imprégnation ne change pas de manière significative la formulation de la matrice. Les résultats sont d'autant meilleurs que Imp est soluble et que le composé $EO_4M$ est fraîchement fabriqué.

Les procédés généraux de fabrication de $EO_4M$ sont en eux-mêmes connus, même si certain composé $EO_4M$ peuvent être nouveau.

Ainsi, selon un procédé avantageux de synthèse du composé de formule I,
a) on réalise la synthèse du composé $EO_4M$ ;
puis de préférence sans séparer $EO_4M$ du milieu réactionnel :
b) on introduit l'imprégnant dans le milieu réactionnel ;
c) on sépare l'éventuel liquide résiduel d'avec le solide réactionnel ;
d) on sèche et éventuellement calcine .

Si l'on se réfère aux techniques générales de fabrication de phosphates [cf "PASCAL P. Nouveau traité de chimie minérale, tome X (1956), pp.821-823 et "GMELINS HANDBUCH der anorganischen chemie (8ième édition)vol.16 (C), pp. 202-206 (1965)], on peut distinguer deux voies principales d'accès aux phosphates. D'une part, la précipitation d'un sel soluble du métal (chlorure, nitrate) par l'hydrogénophosphate d'ammonium; d'autre part, la réaction de l'oxyde de métal avec de l'acide phosphorique à chaud et éventuellement un traitement de finition avec un hydroxyde alcalin.

Dans le cadre de la présente invention, la deuxième procédure permet d'introduire et d'imprégner avantageusement le cation de l'hydroxyde employé dans la phase définition, dans le produit final (I).

Selon la présente invention, ces composés chimiques peuvent être utilisés comme catalyseurs de solvolyse d'un composé halogéné. Ce nouveau type de catalyseur est constitué au moins partiellement d'un composé selon l'invention ci-dessus.

Les catalyseurs selon la présente invention sont tels que la surface des corps catalytiques soit formée pour partie au moins d'un composé selon la présente invention.

Il a été constaté de manière surprenante que les premières minutes ou dizaine de minutes donnaient des résultats moyens en ce qui concerne la sélectivité de la solvolyse. Ce n'est qu'après que le catalyseur aura été modifié par le passage des réactifs pendant une durée pouvant varier d'une dizaine de minutes jusqu'à 2 heures dans les conditions de la solvolyse que le catalyseur jouera pleinement son rôle de catalyseur sélectif. La phase catalytique peut être utilisée pure ou être supportée. Dans la suite de la description, on désignera par corps catalytique le catalyseur dans sa forme particulaire unitaire, que le catalyseur soit supporté ou non.

Le dépôt de la phase catalytique sur le support est faite selon des techniques connues de l'homme de métier.

Ces corps catalytiques peuvent être de toute forme en elles-mêmes connues pour des catalyseurs solides utilisables en phase gazeuse.

Le reste dudit corps catalytique, c'est-à-dire pour l'essentiel la partie n'entrant pas en contact avec le mélange gazeux réactionnel peut être en matériau(x) quelconque(s), pourvu qu'il soit inerte dans les conditions d'usage ; pour des raisons de facilité de fabrication, il peut être réalisé en composés choisis dans le groupe constitué par les phosphates, les hydrogénophosphates et leur mélange. Les catalyseurs peuvent être également réalisés entièrement en composés chimique (I) selon la présente invention.

La surface specifique du catalyseur est la plus élevée possible en général au moins égal à 1 m², avantageusement au moins égal à 10 m², le plus souvent entre 10 et 100 m²/g. cette surface est conditionnée par celle de la matrice.

La présente invention vise également un procédé de solvolyse utilisant les catalyseurs ci-dessus. Ce procédé vise plus particulièrement les halogénures (ou équivalents) d'alcoyle où le terme alcoyle répond à la définition donnée dans le dictionnaire de la Chimie DUVAL "Presse Scientifique, Paris VIe, 1959".

La réaction de solvolyse a de préférence lieu en phase gazeuse.

De préférence le radical alcoyle correspondant à l'halogénure d'alcoyle présente une ou plusieurs des caractéristiques suivantes :

. il est électroattracteur,

. il ne porte pas d'hydrogène sur le(s) carbone(s) en position vicinale de celui qui porte le groupe partant Y,

. il porte sur le(s) dit(s) carbone(s) vicinal des halogènes ou des groupes assimilés, par exemple $CF_3$,

. il est stable dans les conditions opératoires.

Avantageusement, ledit halogénure d'alcoyle répond à la formule (III)

$$R - CX_2 - CH_2Y \quad (III)$$

– où les X sont choisis indépendamment parmi les halogènes de préférence parmi le fluor et le chlore,

– où Y est un groupe partant de préférence le fluor, le chlore, le brome ou l'iode,

– où R est un alcoyle, de préférence électroattracteur, ou un halogène de préférence chlore ou fluor.

Les X sont souvent identiques.

R est avantageusement choisi de manière que le substrat soit volatil à la température de réaction (solvolyse ou élimination de HX pour donner R-CX=CHY). Par volatile, on entend que le substrat présente une température d'ébullition sous 1/10e d'atmosphère ($10^4$ Pa) de préférence sous une atmosphère ($10^5$) inférieure à la température réactionnelle. Ainsi, le nombre de carbones est en général inférieur à 50, avantageusement à 25, de préférence à 10. Bien entendu, R peut être substitué et notamment au moins partiellement fluoré voire perfluoré. Ainsi, en général le nombre de carbone est inférieur à cinquante et même à 25.

Avantageusement dans la formule (III) R représente un fluor.

Dans cette même formule (III) les deux X représentent également avantageusement des atomes de fluor.

Le groupe partant Y, couramment halogène, est en général le chlore et le brome, de préférence, pour des raisons économiques, le chlore.

En revanche, dans le cas de la solvolyse et pour une sélectivité élevée de cette dernière, le fluor est particulièrement intéressant. Toutefois, toutes choses égales par ailleurs, cette selectivité est contrebalancée dans la plupart des cas par un taux de transformation (c'est-à-dire une activité plus faible du catalyseur) moins élevée que pour les autres halogènes.

La température de solvolyse, comme celle de l'élimination, est en général entre 200 et 800°C (dans la présente description, sauf si cela est précisé autrement, les zéros de position ne sont pas des chiffres significatifs), de préférence entre 400 et 600° C.

Pour obtenir de bons résultats, le débit catalytique exprimé en grammes de substrats (par heure) par grammes de catalyseurs est compris entre 0,05 et 10, de préférence entre 0,5 à 5 h⁻¹.

La densité apparente du catalyseur est en général comprise entre 0,3 et 2 de préférence entre 0,8 et 1,5.

Le gaz vecteur est optionnel et est en général un gaz ou un mélange de gaz non réactif dans les conditions opératoires (e.g. $N_2$, air, $H_2$, He et les gaz rares, $N_2$, $H_2$ sont préférés). De préférence le rapport volumique avec le substrat varie de $10^{-2}$ à 50, avantageusement 0,5 à 30, de préférence 0,5 à 10.

Pour ce qui est de la solvolyse, quoiqu'il puisse être envisagé de réaliser la réaction à des pressions inférieures à la pression atmosphérique, il a été trouvé de manière surprenante que l'on avait intérêt à travailler à une pression supérieure à la pression normale avantageusement entre 1 et 100 atmosphères ($10^5$ à $10^7$ Pa) de préférence de 1 à 20 ($10^5$ à $2.10^6$ Pa).

Avantageusement, le rapport molaire entre solvants et substrats, compris entre 1 et 100, de préférence entre 2 et 10.

Les solvants utilisés pour la solvolyse sont de préférence des réactifs nucléophiles polaires protiques ne donnant pas de réaction parasite significative dans les conditions opératoires.

Ils sont avantageusement volatils dans les conditions opératoires ; par exemple leur pression de vapeur saturante à la température de réaction est avantageusement au moins égale à 1 d'atmosphère ($10^5$ Pa), de préférence $10^6$ Pa.

Parmi les solvants donnant les meilleurs résultats, il convient de citer les amines y compris les anilines, primaires ou secondaires et les alcools primaires, secondaires ou tertiaires, y compris les phénols.

L'eau donne également des résultats particulièrement bons.

Pour les opérations de formation d'insaturé il est possible de réduire, voire de porter à zéro, la quantité de solvant (l'eau dans ce cas), pour favoriser cette formation. en revanche pour favoriser la solvolyse, qui semble la réaction la plus difficile on peut pallier une selectivité insuffisante en jouant sur le rapport molaire entre solvants et substrats, voire en l'augmentant encore par rapport aux valeurs ci-dessus.

Les exemples non limitatifs suivants illustrent l'invention.

## Protocole opératoire des exemples de catalyse plus spécialement adapté à la solvolyse

– l'ensemble est chauffé pendant une demi-heure à la température de réaction choisie,

– puis on fait circuler dans le tube (en rentrant du côté où se trouve le lit de billes en verre) le mélange des réactifs gazeux (solvant et chloro-1 trifluoro-2,2,2 éthane). On piège les gaz sortant du réacteur et on procède par chromatographie en phase gazeuse à l'analyse des produits obtenus (produits dont la structure est confirmée par spectrométrie de masse). Les mesures rapportées ici sont celles qui correspondent à l'état permanent.

La sélectivité (RT) est définie comme étant la quantité en mole(s) de substrat de TFE obtenu par rapport à la quantité en (moles) de chloro-1 trifluoro-2,2,2 éthane transformé.

Le rapport RR correspond au rapport entre la quantité de moles transformées en produit désiré et la quantité initiale de substrat lors de la réaction.

TT correspond au rapport entre le substrat transformé et le substrat initial.

## PROTOCOLE DES TESTS DE CATALYSE

Les réactions sont effectuées dans un réacteur phase vapeur, lit fixe tubulaire (L = 200 mm diamètre = 15 mm) en quartz.

L'azote et le substrat en général le flugène ($CF_3$-$CH_2Cl$) sont introduits par les débimètres volumiques vendus sous la dénomination commerciale "Brooks" (la quantité exacte de flugène introduit est déterminée par pesée, avant et après réaction de la bouteille de flugène).

L'eau est introduite à l'aide d'une seringue actionnée par un pousse seringue.

Le réacteur est placé dans un four à coquille équipé d'une régulation de température.

. Déroulement de l'essai :

. Chargement du catalyseur,

. Chargement d'un lit de billes de verre sur le catalyseur (zone de préchauffage et de prémélange des réactifs),

. Chauffage à la température de réaction (indifféremment sous air ou sous gaz inerte comme l'azote),

. Equilibre thermique du réacteur (1 heure),

. Injection des réactifs jusqu'à établissement de l'équilibre permanent (en général 30 minutes) (traitement de conditionnement pour obtenir une bonne sélectivité),

. Piégeage des vapeurs issues de la réaction (pièges contenant du n-propanol),

. Analyse.

## EXEMPLES LIMINAIRES :

### tests 1-6 : Préparations de catalyseurs

### Mode opératoire général pour du $LaPO_4$ dopé

De l'oxyde métallique $La_2O_3$ (0,5 mole) est ajouté en 30 minutes à une solution de $H_3PO_4$ 86 % (1 mole) dans de l'eau distillée (300 cm$^3$) à une température de 90° C et avec une bonne agitation. On ajoute une solution aqueuse d'hydroxyde alcalin ou alcalino-terreux (0,2 mole) jusqu'à obtention de la neutralité.

La suspension est agitée à 90 °C pendant 20 minutes. Le solide est récupéré par filtration, lavé avec de l'eau distillée, séché à 100 °C sous 200 torr, pendant 16 heures et calciné à 500° C pendant 3 heures sous air.

7

Les données fournies par l'analyse élémentaire sont résumées dans le tableau 1.

Tableau 1 . Synthèse de "LaPO₄, M"

| TEST | "DOPANT" M | Analyses élémentaires(%) | | | Rapport atomique | |
|---|---|---|---|---|---|---|
| | | La | P | M | M/La * | La/M |
| 1 | Li | 54,02 | 11,5 | 0,34 | 0,13 | 7,7 |
| 2 | Na | 53,9 | 11,3 | 0,9 | 0,10 | 10 |
| 3 | K | 53,95 | 11,3 | 1,25 | 0,08 | 12,5 |
| 4 | Cs | 55,0 | 11,4 | 3,5 | 0,06-0,07 | 15,4 |
| 5 | Sr | 53,6 | 12,6 | 4,0 | 0,12 | 8,3 |
| 6 | Ba | 51,1 | 11,7 | 5,6 | 0,12 | 8,3 |

## TEST DES CATALYSEURS

On utilise le protocole spécifié ci-dessus.
. Conditions expérimentales :
    . Température : 490°C,
    . Débit de flugène : 46 mmol/h = 5,45g/h,
    . Débit $H_2O$ : 230 mmol/h = 4,14g/h,
    . Débit $N_2$ : 1,05 1/h,
    . Catalyseur :$LaPO_4$,(CsOH) obtenu lors de la synthèse N°4(cf tableau).
    3 ml de poudre (4,4 g) dispersés dans 5 ml de quartz en grains, (diamètre moyen 0,6 mm),
    – Rapport volumique : $H_2O$/F133 = 5
    – Débit catalytique = 1,24 $H^{-1}$
Résultats :

TT flugène 133 = 22 %
RR Trifluoroéthanol = 19,9 %
RT Trifluoroéthanol = 90 %
Les essais préliminaires sur les catalyseurs 1,2,3,5,6 montrent qu'ils présentent tous une activité de catalyse. Il semble en outre que les alcalins soient meilleurs que les alcalino-terreux
Alt = Sr ; TT = 19 % ; RR = 4 % ; RT = 21 % ;
Alt = Ba ; TT = 25 % ; RR = 3,3 % ; RT = 13 % ;
et que la sélectivité augmente avec le nombre atomique.

### Généralités sur la synthèse des phosphates selon l'invention

Les phosphates mixtes selon l'invention, notamment les phosphates de terres rares et les phosphates de terres rares et d'alcalins peuvent être préparés par voie humide ou voie sèche. La voie humide est en général préférée pour la mise en oeuvre de la présente invention. Ainsi ils peuvent être préparés par :
    – Réaction dite solide-solide d'un mélange mécanique de sels de cations dérivés et de phosphate. Le mélange est porté à une température supérieure à la température de fusion la plus haute des sels présents. La réaction peut être menée dans un milieu ouvert, ou dans un milieu fermé en atmosphère inerte ou non, suivant qu'il y a dégagement de gaz ou pas ;
    – Co-précipitation en milieu aqueux, ammoniac ou organique d'un mélange des sels de cations dérivés et d'une source de phosphate.
La réaction peut être menée à une température inférieure à 100°C dans un réacteur classique ouvert ou fermé, balayé ou non par un gaz inerte. Elle peut être également menée à une température supérieure à 100°C dans un autoclave avec ou sans agitation.

L'ordre d'ajout des sels de cations et de la source de phosphate n'est pas critique.

On peut d'abord mélanger les sels de cations dans une solution, puis ajouter la source de phosphate ou l'inverse.

La précipitation commence dès que le phosphate est ajouté.

On peut également précipiter préalablement le phosphate de terres raresavant d'ajouter dans le milieu réactionnel le sel d'alcalin.

On peut également mélanger préalablement le sel d'alcalin et la source de phosphate, puis ajouter à ce mélange le sel de terres rares.

Le mélange de sels de cations, ainsi que la précipitation par ajout de la source de phosphate peuvent se faire à froid ou à chaud. Si la précipitation se fait à froid, elle peut être suivie d'un mûrissement à chaud, en réacteur ouvert pour une température au plus égale à 100°C, en autoclave au-delà.

La réaction de précipitation peut se faire à n'importe quel pH entre pH de 0,5 et pH 13. Elle est conduite préférentiellement entre pH 1 et pH 10 et plus préférentiellement entre pH 1 et pH 6.

Le pH du milieu est fonction de la nature des sels de cation et de la source de phosphate choisi et peut être également ajusté par un acide minéral (HCl, $NO_3$) ou organique ou'une base minérale (NaOH, CsOH, $NH_4OH$), ou organique (amine, acide benzoïque, acide oxalique...) à la valeur souhaitée avant ou en cours de précipitation.

Le pH peut être également variable en cours de précipitation :

– soit par variation naturelle lors de l'ajout d'un des réactifs dans l'autre, le pH variant alors du domaine basique vers le domaine acide ou l'inverse ;

– soit par ajout d'une base dite retard (il s'agit en fait d'un composé précurseur d'une base, composé dont la thermolyse libère une base vraie. On peut citer comme exemple de la base retard l'urée, qui dégage par thermolyse de l'ammoniac(que)...) dans le milieu réactionnel acide.

Dans ce dernier cas le mélange des sels de cation, de la source de phosphate et de la base retard se fait à pH suffisamment bas pour empêcher toute précipitation.

On chauffe le milieu réactionnel et fait monter le pH par décomposition thermique du précurseur retard de la base entraînant une précipitation homogène.

La réaction de précipitation pourra se faire éventuellement en présence d'agent organique de type ammonium quaternaire de formule générale $R_4N^+X^-$ où R est un alkyle à un carbone au moins et $X^-$ est un anion indifférent tel que halogénure ou un hydroxyde.

La réaction de co-précipitation peut se faire avec ou sans agitation, mais sera faite préférentiellement avec une agitation.

Les produits ainsi obtenus peuvent être lavés ou non avec une solution aqueuse acide, neutre ou basique, le pH de cette solution étant controlé par un acide ou une base minérale (HCl, $NH_4OH$, NaOH) ou organique (acide acétique, hydroxyde de tétrapropyl ammonium...).

Le lavage peut également se faire avec un solvant organique : méthanol, toluène, etc...

Le lavage peut se faire par remise en suspension dans l'eau ou par filtration sur un filtre.

Le produit obtenu après lavage peut être séché ou non. Le séchage se fait avantageusement à 110°C environ sous atmosphère normale, ou sous vide partiel ou par lyophilisation.

Il peut également être calciné jusqu'à 900°C pendant un temps s'étalant de une heure à 10 heures (préférentiellement pas au-delà de 500°C).

Le produit ainsi obtenu peut être également chimiquement modifié par imprégnation à sec ou voie humide. L'imprégnation à sec consiste à ajouter à une masse $m_1$ d'une poudre du produit à imprégner un volume V d'une solution aqueuse d'un ou des sels de cations ou d'anions à fixer sur la surface du solide. Le volume V de solution est choisie tel que $V/m_1$ soit égal au volume poreux à l'eau du solide à imprégner.

La concentration C en cations ou anions de la solution imprégnante est choisie telle que le rapport $CVM_2/m_1$ soit égal au pourcentage en poids choisi d'espèce imprégnante fixée sur la surface du produit à imprégner (avec $M_2$ = masse molaire de l'espèce imprégnante). On procède goutte à goutte à l'ajout de la solution de manière à obtenir une adsorption homogène.

Le produit peut ensuite être laissée au repos pendant un temps variable à température ambiante. Le produit est ensuite séché suivant les techniques classiques connues de l'homme de l'art et indiquées brièvement ci-dessus.

Le produit peut être ensuite calciné à au moins 500°C pendant 2 heures ou servir directement de catalyseur, la calcination aura alors lieu à la température et dans les conditions de la réaction.

L'imprégnation voie humide se fait en redispersant le solide obtenu par réaction solide-solide ou co-précipitation dans une solution aqueuse de sels de cations et/ou d'anions à fixer sur la surface du solide.

Cette solution peut présenter une concentration variant de $10^{-3}$M à 10 M en l'espèce imprégnante.

Le pH de la solution pourra être avantageusement ajusté à une valeur au moins égale à celle du point isoélectrique du produit à modifier pour fixer préférentiellement les cations (cas usuel) ; toutefois cette condition n'est pas impérative il est possible, au-dessous de ce point isoélectrique, de fixer correctement les cations lorsque les anions associés ont un caractère très"covalent" comme les sulfates et les phosphates.

La température de la solution peut varier de l'ambiante à 100°C. La dispersion est agitée vigoureusement, pendant un temps variable. Le produit est ensuite filtrée et éventuellement lavée avec les techniques décrites précédemment.

Dans les exemples qui suivent le produit est ensuite séché comme indiqué précédemment et calciné à au moins 500°C pendant au moins 2 heures.

## Exemple N° 1

On introduit 57g d'$H_3PO_4$ (85 % ; d=1,7 ; PROLABO) et 150 ml d'eau dans un tricol de 1 litre.

On agite à 500-700 tours/minute. On chauffe jusqu'à atteindre 90°C.

On ajoute 80,5g de La2O3 (PROLABO) par portions, en 30 à 40 minutes sous agitation (2 à 3 spatules à la fois toutes les 4 à 5 minutes).

On maintient la température entre 87 et 93°C.

Après addition, on arrête l'agitation pour rincer les parois (50 à 70 g d'eau). On agite à nouveau (500-700 tours/minute) pendant 1h15 à 90°C.

On prépare une solution de NaOH en diluant dans 25 ml d'eau une masse de NaOH prolabo (en la supposant sans carbonate) correspondant à 125 mmoles de NaOH.

On ajoute cette solution à la suspension sous agitation (500 à 700 tours/minute) en 20 à 30 secondes en continu.

On arrête l'agitation et rince les parois (50 à 70g d'eau).

On agite à nouveau (500 à 700 tours/minute) et chauffe 1h15 à 90°C.

On laisse refroidir jusqu'à température ambiante sous agitation pendant une demi-heure et on finit dans un bain d'eau froide. La suspension est épaisse.

On filtre en versant la suspension sur un fritté n°3 (d= 130) jusqu'à épuisement des eaux-mères.

Le produit reste humide. On lave en ajoutant 250 ml d'eau sur le fritté en agitant avec une spatule (plastique ou éventuellement en inox) : la suspension paraît homogène. On recommence l'opération 2 autres fois.

A chaque étape, la filtration se fait jusqu'à épuisement des eaux de lavages. On récupère 1000-1100 ml d'eaux mères et d'eaux de lavages.

Le pH minimum des eaux récupérées est de 9.

La durée totale de la synthèse est d'environ une journée. La gâteau laissé sur fritté est séché une nuit à 100°C sous vide (60 mm Hg). Le produit obtenu est aisément broyé et ramené à la taille de particule souhaitée. On calcine le solide 2h30 à 500°C sous air ambiant et on laisse refroidir à température ambiante sous air.

On a déterminé que la valeur de p exprimée en rapport équivalent-gramme/mole (cf. description) est égale à $8.10^{-2}$.

## Exemple N° 2

On procède comme à l'exemple 1, en remplaçant NaOH par KOH (prolabo).

On a déterminé que la valeur de p exprimée en rapport équivalent-gramme/mole est égale à $8.10^{-2}$.

## Exemple N°3

On procède comme à l'exemple N°1, en remplaçant NaOH par Cs OH, $H_2O$ (prolabo).

On a déterminé que la valeur de p exprimée en rapport équivalent-gramme/mole est égale à $8.10^{-2}$.

## Exemple N°4

On procède comme à l'exemple N°1, en remplaçant NaOH par LiOH (prolabo).

On a déterminé que la valeur de p exprimée en rapport équivalent-gramme/mole est égale à $9.10^{-2}$.

## Exemple N° 5

On procède comme à l'exemple n°3 sauf pour l'étape de neutralisation et de lavage.

a) la neutralisation du milieu réactionnel se fait avec une solution

6M de Cs OH jusqu'à ce que le pH des eaux mères atteigne une valeur de 9, soit après ajout d'environ 45 cc de solution basique.

b) le produit obtenu après filtration ne subit aucun lavage. Le produit est alors séché à 110° C une nuit et calciné à 500°C pendant 2 heures. L'échantillon ainsi obtenu contient 6 % de césium sur sec.

On a déterminé que la valeur de p exprimée en rapport équivalent-gramme/mole est égale à $12,6.10^{-2}$.

Exemple N° 6

On procède comme à l'exemple n°5 jusqu'à l'étape de lavage.

Le produit filtré est alors remis en suspension dans de l'eau permutée, sous agitation pendant une demi-heure à température ambiante. La suspension est telle qu'on a 30 ml d'eau pour l'équivalent en produit humide de 10 grammes de produit séché à 110°C.

La suspension est alors centrifugée.

Le produit est séché à 110°C une nuit et calciné à 500°C pendant 2 heures.

L'échantillon ainsi obtenu contient 3 % en poids de césium sur sec.

On a déterminé que la valeur de p exprimée en rapport équivalent-gramme/mole est égale à $6,1.10^{-2}$.

Exemple N° 7

On procède de la même manière qu'à l'exemple n°6 en renouvellant le lavage 2 fois. Le produit est donc lavé 3 fois, puis séché à 110°C une nuit et calciné à 500°C pendant 2 heures.

Le produit ainsi obtenu contient 1 % en poids de césium sur sec.

On a déterminé que la valeur de p exprimée en rapport équivalent-gramme/mole (cf. description) est égale à $2,1.10^{-2}$.

Exemple N° 8

On introduit 57 g de $H_3PO_4$ (85 %, prolabo) et 150 ml d'eau dans un tricol de 1 litre.

On agite à 500-700 tours minute.

On introduit 166,6 g de $La_2(CO_3)_3$, $12H_2O$ à froid, lentement et sous forte agitation.

On chauffe ensuite le milieu réactionnel jusqu'à 90°C pendant une heure.

On laisse refroidir jusqu'à température ambiante sous agitation pendant une demi-heure et on finit dans un bain d'eau froide.

La suspension est filtré sur un fritté n°3 jusqu'à épuisement des eaux-mères.

Le produit est alors redispersé dans un litre d'eau sous forte agitation et laissé en suspension pendant une demi-heure tout en maintenant l'agitation.

Le produit est à nouveau filtré sur fritté n°3. On recommence cette opération de lavage 2 autres fois.

Le produit est ensuite filtré et séché à 110°C et présente un volume poreux à l'eau de 0,4 cc/g. On l'appellera le produit à imprégner.

On prélève 4,7 cc d'une solution 6M de Cs OH, à laquelle on ajoute 14,12 cc d'une solution 1M de $H_3PO_4$. On ajoute alors la quantité d'eau nécessaire pour compléter à 50 cc.

On prend 50 g de produit à imprégner que l'on met dans un bécher de 200cc.

On introduit goutte à goutte 20 cc de la solution d'imprégnation en écrasant les agglomérats formés et en homogénéisant bien.

On laisse le produit reposer 1 heure. On le sèche une nuit à 110°C et on le calcine 2 heures à 500°C.

Le produit est de structure hexagonale.

Teneur en cesium sur sec = 3 %.

On a déterminé que la valeur de p exprimée en rapport équivalent-gramme/mole est égale à $5,9.10^{-2}$.

Exemple N° 9

On procède comme à l'exemple n°8 avec une solution d'imprégnation différente.

Cette solution est préparée en amenant à 50 cc une solution de 4,7 cc de CsOH 6 M.

La solution ainsi obtenue est 0,564 molaire.

On imprègne alors 50 g du produit à imprégner avec 20 cc de cette solution suivant la procédure indiquée à l'exemple 8. Le produit est ensuite séché à 110°C une nuit et calciné à 500°C pendant 2 heures.

On a déterminé que la valeur de p exprimée en rapport équivalent-gramme/mole est égale à $5,9.10^{-2}$.

### Exemple N° 10

On imprègne 50 g de produit à imprégner avec 20 cc d'une solution 0,188 molaire de $Cs_3LaCl_6$. On procède ensuite comme indiqué à l'exemple n°8.

On a déterminé que la valeur de p exprimée en rapport équivalent-gramme/mole est égale à $5,9.10^{-2}$.

### Exemple N° 11

On imprègne 50 g de produit à imprégner avec 20 cc d'une solution 0,564 molaire de CsF. On procède ensuite comme indiqué à l'exemple N°8.

On a déterminé que la valeur de p exprimée en rapport équivalent-gramme/mole est égale à $5,9.10^{-2}$.

### Exemple N° 12

On imprègne 50 g de produit à imprégner avec 20 cc d'une solution 0,282 molaire de $Cs_2 SO_4$. On procède ensuite comme à l'exemple n°8.

On a déterminé que la valeur de p exprimée en rapport équivalent-gramme/mole est égale à $5,9.10^{-2}$.

### Exemple N° 13

On procède comme dans l'exemple n° 8 avec une solution 0,282 molaire de $Cs_2 SO_3$.

On a déterminé que la valeur de p exprimée en rapport équivalent-gramme/mole est égale à $5,9.10^{-2}$.

### Exemple N° 14

On procède comme dans l'exemple n°8 avec une solution 0,282 molaire de $Cs_2 C_2O_4$.

On a déterminé que la valeur de p exprimée en rapport équivalent-gramme/mole est égale à $5,9.10^{-2}$.

### Exemple N° 15

On procède comme dans l'exemple n° 8 avec une solution 0,564 molaire de benzoate de césium.

On a déterminé que la valeur de p exprimée en rapport équivalent-gramme/mole est égale à $5,9.10^{-2}$.

### Exemple N° 16

On procède au début comme dans l'exemple n° 1.

puis avant la neutralisation avec la base minérale forte, le produit est filtré, sur un fritté n°3 et lavé sur fritté 3 fois avec 250 ml d'eau, en agitant le produit avec une spatule.

Le produit est ensuite séché à 110°C une nuit.

On redisperse ensuite le produit sous agitation dans un litre d'eau distillée. On ajoute une solution 6 N de CsOH jusqu'à ce que le pH de la solution soit de 9, toujours sous forte agitation et à température ambiante.

Le produit est alors filtré sur fritté n°3 et lavé 3 fois sur fritté avec 250 ml d'eau distillée.

Le produit est ensuite séché à 110°C une nuit et calciné 500°C pendant 2 heures.

### Exemple N° 17

On prépare 0,75 l d'une solution (A) 0,5 molaire de $La(NO_3)_3$ et 0,5 litre d'une solution (B) 0,75 molaire de $(NH_4)_2 HPO_4$.

Les solutions A et B sont mises à chauffer séparément à 80°C.

On ajoute alors en une demi-heure la solution B dans la solution A sous forte agitation et on porte ensuite la température à 90°C. On reste une heure à cette température. On laisse la température baisser à 80°C. On ajoute alors dans le milieu réactionnel 75 cc d'une solution 6M de CsOH.

On refroidit le mélange et on centrifuge la suspension.

Le produit est alors redispersé sous agitation dans 1000 cc d'eau distillé et filtré.

On procède ainsi à 3 lavages.

Le produit est ensuite séché à 110°C.

Le volume poreux à l'eau du produit ainsi obtenu est de 1 cc/g.

On prépare 50 cc d'une solution de CsOH et $H_3PO_4$ à partir de 3,76 cc de CsOH 3M et 5,64 cc de $H_3PO_4$

**1M.**

On procède comme décrit dans l'exemple n°8, mais avec 10 cc de solution pour 10 g de produit.

On a déterminé que la valeur de p exprimée en rapport équivalent-gramme/mole est égale à $5,9.10^{-2}$.

### Exemple N° 18

Le produit séché à 110° C une nuit après imprégnation obtenu à l'exemple n°8 est calciné à 700°C pendant 2 heures. Ce produit est de structure monoclinique.

On a déterminé que la valeur de p exprimée en rapport équivalent-gramme/mole est égale à $5,9.10^{-2}$.

### Exemple N° 19

Le produit séché à 110°C avant imprégnation de l'exemple n°8 appelé produit à imprégner est calciné à 700°C pendant 2 heures.

La structure passe ainsi de la phase hexagonale à la phase monoclinique.

Le produit a une volume poreux de 0,28 cc/g.

On prépare une solution de 100 cc de CsOH et $H_3PO_4$ à partir de 22,03 cc de CsOH 1,5 M et 16,52 cc de $H_3PO_4$ 1M.

On procède à l'imprégnation de 10 g du produit monoclinique avec 2,8 cc de la solution ainsi préparée.

Le produit est ensuite séché à 110°C une nuit et calciné 500°C pendant 2 heures.

On a déterminé que la valeur de p exprimée en rapport équivalent-gramme/mole est égale à $2,1.10^{-2}$.

### Exemple N° 20

On reproduit la synthèse décrite dans l'exemple n°1 en substituant $La_2O_3$ par $Sm_2O_3$, (mole pour mole) et ceci jusqu'avant l'étape de neutralisation.

Le produit est alors filtré, lavé sur fritté 3 fois et séché à 110°C une nuit.

Il a un volume poreux de 0,35 cc/g.

On imprègne alors 10 g du produit avec 3,5 cc des 50 cc d'une solution de CsOH et $H_3PO_4$ préparée à partir de 5,16 cc de CsOH 3M et 7,74 cc de $H_3PO_4$ 1M, suivant la procédure de l'exemple n°8.

On a déterminé que la valeur de p exprimée en rapport équivalent-gramme/mole est égale à $3.10^{-2}$.

### Exemple N° 21

Il s'agit d'un solide de type hydrogéno-phosphate de calcium de grande pureté ($CaH\,PO_4$, $nH_2O$), de 30 m2/g de surface environ. Ce produit est imprégné selon la procédure de l'exemple 8.

### Exemple N° 22

Dans un réacteur tubulaire, on place 5g de solide préparé selon l'exemple 1. L'ensemble réacteur et catalyseur est chauffé à 490°C pendant 1/2 heure. On fait ensuite circuler sur le solide le mélange des réactifs gazeux : eau et chloro-1-trifluoro-2,2,2 éthane. Ceux-ci sont en mélange avec de l'azote dans le rapport molaire $H_2O/N_2/CF_3CH_2Cl$ = 5/1/1. Le flux gazeux à la température réactionnelle est de 18 l/h. A la sortie du réacteur les gaz sont piégés et sont analysés par chromatographie en phase gazeuse.

Après une période d'environ 1/2 heure (permettant d'atteindre l'équilibre réactionnel) les performances suivantes sont observées :

Conversion du chloro-1 trifluoro-2,2,2 éthane : 18,6 %.

Sélectivité en trifluoroéthanol : 38 %.

Sélectivité en chloro-1 difluoro-2,2 éthylène : 10,4 %.

### Exemple N° 23

Le protocole de l'exemple 22 est repris en utilisant 5 g de solide préparé selon l'exemple 2.

Conversion du chloro-1 trifluoro-2,2,2 éthane : 26 %.

Sélectivité en trifluoroéthanol : 53 %.

Sélectivité en chloro-1 difluoro éthylène : 13 %.

Exemple N° 24

L'utilisation suivant le protocole de l'exemple 22 du matériau préparé selon l'exemple 3 conduit aux performances suivantes :
Conversion du chloro-1 trifluoro-2,2,2 éthane : 26 %.
Sélectivité en trifluoroéthanol : 90 %.
Sélectivité en chloro-1 difluoroéthylène : 6,4 %.

Exemple N° 25

Le protocole de l'exemple 22 est repris à l'aide du matériau préparé selon l'exemple 4 :
Conversion du chloro-1 trifluoro-2,2,2 éthane : 6 %.
Sélectivité en trifluoroéthanol : 38 %.
Sélectivité en chloro-1 difluoroéthylène : 37 %.

Exemple N° 26

Selon l'exemple 22 par utilisation du solide préparé selon l'exemple 5, on obtient :
Conversion du chloro-1 trifluoro-2,2,2 éthane : 26,8 %.
Sélectivité en trifluoroéthanol : 87,7 %.
Sélectivité en chloro-1 difluoroéthylène : 7,5 %.

Exemple N° 27

Selon l'exemple 22, par utilisation du solide préparé selon l'exemple 6 on obtient :
Conversion du chloro-1 trifluoro-2,2,2 éthane : 20,6 %.
Sélectivité en trifluoroéthanol : 93 %.
Sélectivité en chloro-1 difluoroéthylène : 7 %.

Exemple N° 28

Selon l'exemple 22, par utilisation du solide préparé selon l'exemple 7, on obtient :
Conversion du chloro-1 trifluoro-2,2,2 éthane : 8,5 %.
Sélectivité en trifluoroéthanol : 88,3 %.
Sélectivité en chloro-1 difluoroéthylène : 11,7 %.

Exemple N° 29

Selon l'exemple 22, par utilisation du solide préparé selon l'exemple 8, on obtient :
Conversion du chloro-1 trifluoro-2,2,2 éthane : 23,7 %.
Sélectivité en trifluoroéthanol : 90,7 %.
Sélectivité en chloro-1 difluoroéthylène : 9,3 %.

Exemple N° 30

Dans un réacteur tubulaire, on place 5g de solide préparé selon l'exemple 8. L'ensemble réacteur et catalyseur est chauffé à 435° C pendant 1/2 h. On fait ensuite circuler sur le solide le mélange des réactifs gazeux: eau et chloro-1 trifluoro-2,2,2 éthane. Ceux-ci sont en mélange avec de l'azote dans le rapport molaire $H_2O/N_2 / CF_3CH_2Cl = 5/1/1$. Le flux gazeux à la température réactionnelle est de 10 l/h. A la sortie du réacteur les gaz sont piégés et sont analysés par chromatographie en phase gazeuse :
Conversion du chloro-1 trifluoro-2,2,2 éthane : 10,6 %.
Sélectivité en trifluoroéthanol : 95 %.

Exemple N° 31

Dans un réacteur tubulaire, on place 5 g de solide préparé selon l'exemple 8. L'ensemble réacteur et catalyseur est chauffé à 470°C pendant 1/2 h. On fait ensuite circuler sur le solide le mélange des réactifs gazeux: eau et chloro-1 trifluoro-2,2,2 éthane. Ceux-ci sont en mélange avec de l'azote dans le rapport molaire

**14**

$H_2O/N_2/CF_3CH_2Cl$ = 3/1/1.

Le flux gazeux à la température réactionnelle est de 13l/h.

A la sortie du réacteur, les gaz sont piégés et analysés par chromatographie en phase gazeuse :

Conversion du chloro-1 trifluoro-2,2,2 éthane : 19,1 %.

Sélectivité en trifluoroéthanol : 93,2 %.

Sélectivité en chlorodifluoroéthylène : 6,8 %.

Exemple N° 32

Selon l'exemple 22, par utilisation du solide préparé selon l'exemple 9, on obtient :

Conversion du chloro-1 trifluoro-2,2,2 éthane : 23 %.

Sélectivité en trifluoroéthanol : 87 %.

Sélectivité en chlorodifluoroéthylène : 13 %.

Exemple N° 33

Selon l'exemple 22, par utilisation du solide préparé selon l'exemple 10, on obtient :

Conversion du chloro-1 trifluoro-2,2,2 éthane : 12 %.

Sélectivité en trifluoroéthanol : 60 %.

Sélectivité en chloro-1 difluoro-2,2 éthylène : 40 %.

Exemple N° 34

Selon l'exemple 22, par utilisation du solide préparé selon l'exemple 11, on obtient :

Conversion du chloro-1 trifluoro-2,2,2 éthane : 18,5 %.

Sélectivité en trifluoroéthanol : 82 %.

Sélectivité en chloro-1 difluoro-2,2 éthylène : 18 %.

Exemple N° 35

Selon l'exemple 22, par utilisation du solide préparé selon l'exemple 12, on obtient :

Conversion du chloro-1 trifluoro-2,2,2 éthane : 19,5 %.

Sélectivité en trifluoroéthanol : 89 %.

Sélectivité en chloro-1 difluoro-2,2 éthylène : 11 %.

Exemple N° 36

Selon l'exemple 22, par utilisation du solide préparé selon l'exemple 13, on obtient :

Conversion du chloro-1 trifluoro-2,2,2 éthane : 21 %.

Sélectivité en trifluoroéthanol : 81 %.

Sélectivité en chloro-1 difluoro-2,2 éthylène : 19 %.

Exemple N° 37

Selon l'exemple 22, par utilisation du solide préparé selon l'exemple 14, on obtient :Conversion du chloro-1 trifluoro-2,2,2 éthane : 21,5 %.

Sélectivité en trifluoroéthanol : 66 %.

Sélectivité en chloro-1 difluoro-2,2 éthylène : 15 %.

Exemple N° 38

Selon l'exemple 22, par utilisation du solide préparé selon l'exemple 15, on obtient :

Conversion du chloro-1 trifluoro-2,2,2 éthane : 21 %.

Sélectivité en trifluoroéthanol : 80 %.

Sélectivité en chloro-1 difluoro-2,2 éthylène : 20 %.

Exemple N° 39

Selon l'exemple 22, par utilisation du solide préparé selon l'exemple 16, on obtient :
Conversion du chloro-1 trifluoro-2,2,2 éthane : 26 %.
Sélectivité en trifluoroéthanol : 68 %.
Sélectivité en chloro-1 difluoro-2,2 éthylène : 10 %.

Exemple N° 40

Selon l'exemple 22, par utilisation du solide préparé selon l'exemple 17, on obtient :
Conversion du chloro-1 trifluoro-2,2,2 éthane : 16,4 %.
Sélectivité en trifluoroéthanol : 86,5 %.
Sélectivité en chloro-1 difluoro-2,2 éthylène : 11,2 %.

Exemple N° 41

Selon l'exemple 22, par utilisation du solide préparé selon l'exemple 18, on obtient :
Conversion du chloro-1 trifluoro-2,2,2 éthane : 24,1 %. Sélectivité en trifluoroéthanol : 91,7 %.
Sélectivité en chloro-1 difluoro-2,2 éthylène : 8,3 %.

Exemple N° 42

Selon l'exemple 22, par utilisation du solide préparé selon l'exemple 19, on obtient :
Conversion du chloro-1 trifluoro-2,2,2 éthane : 9 %.
Sélectivité en trifluoroéthanol : 39 %.
Sélectivité en chloro-1 difluoro-2,2 éthylène : 19 %.

Exemple N° 43

Selon l'exemple 22, par utilisation du solide préparé selon l'exemple 20, on obtient :
Conversion du chloro-1 trifluoro-2,2,2 éthane : 4 %.
Sélectivité en trifluoroéthanol : 95 %.

Exemple N° 44

Selon l'exemple 22, par utilisation du solide préparé selon l'exemple 21, on obtient :
Conversion du chloro-1 trifluoro-2,2,2 éthane : 14 %.
Sélectivité en trifluoroéthanol : 31 %.
Sélectivité en chloro-1 difluoro-2,2 éthylène : 16,4 %.

Exemple N° 45

Dans un réacteur tubulaire, on place 5 g de solide préparé selon l'exemple 1. L'ensemble réacteur et catalyseur est chauffé à 490°C pendant 1/2 heure. On fait ensuite circuler sur le solide le mélange des réactifs gazeux : eau et bromo-1 trifluoro-2,2,2 éthane. Ceux-ci sont en mélange avec de l'azote dans le rapport molaire $H_2O/N_2/CF_3Br = 5/1/1$.

Le flux gazeux à la température réactionnelle est de 18 l/h. A la sortie du réacteur, les gaz sont piégés et sont analysés par chromatographie en phase gazeuse.

Après une période d'une heure permettant d'atteindre l'équilibre réactionnel, les performances suivantes sont observées :
Conversion du bromo-1 trifluoro-2,2,2 éthane : 5,6 %.
Sélectivité en trifluoroéthanol : 83,9 %.
Sélectivité en bromo-1 difluoro-2,2 éthylène : 16,1 %.

Exemple N° 46

Selon l'exemple 45, par utilisation du solide préparé dans l'exemple 2, on obtient :
Conversion du bromo-1 trifluoro-2,2,2 éthane : 17,4 %.

16

Sélectivité en trifluoroéthanol : 94,8 %.
Sélectivité en bromo-1 difluoro-2,2 éthylène : 5,2 %

## Exemple N° 47

Selon l'exemple 45, par utilisation du solide préparé selon l'exemple 3, on obtient :
Conversion du bromo-1 trifluoro-2,2,2 éthane : 23,4 %.
Sélectivité en trifluoroéthanol : 95,7 %.
Sélectivité en bromo-1 difluoro-2,2 éthylène : 4,3 %.

## Exemple N° 48

Dans un réacteur tubulaire, on place 5 g de solide préparé selon l'exemple 3. L'ensemble réacteur et catalyseur est chauffé à 490°C pendant 1/2 heure. On fait ensuite circuler sur le solide le mélange des réactifs gazeux : eau et tétrafluoro-1,2,2,2 éthane. Ceux-ci sont en mélange avec de l'azote dans le rapport molaire $H_2O/N_2/CF_3CH_2F = 5/1/1$. Le flux gazeux à la température réactionnelle est de 18 l/h. A la sortie du réacteur, les gaz sont piégés et sont analysés par chromatographie en phase gazeuse.

Après une période d'une heure permettant d'atteindre l'équilibre réactionnel, les performances suivantes sont observées : Conversion du tétrafluoro-1,2,2,2 éthane : 11 %.

Sélectivité en trifluoroéthanol : > 95 %.

## Exemple N° 49

Selon l'exemple 48, par utilisation du solide préparé selon l'exemple 2, on obtient :
Conversion du tétrafluoro-1,2,2,2 éthane : 8,5 %.
Sélectivité en trifluoroéthanol : > 95 %.

## Exemple N° 50

Selon l'exemple 48, par utilisation du solide préparé selon l'exemple 1, on obtient :
Conversion du tétrafluoro-1,2,2,2 éthane : 5 %.
Sélectivité en trifluoroéthanol : > 95 %.

## Exemple N° 51

Dans un réacteur tubulaire, on place 5g de solide préparé selon l'exemple 8. L'ensemble réacteur et catalyseur est chauffé à 490°C pendant 1/2h. On fait ensuite circuler sur le solide le mélange des réactifs gazeux: ammoniaque et chloro-1 trifluoro-2,2,2 éthane. Ceux-ci sont en mélange avec de l'azote dans le rapport molaire $NH_3/N_2/CF_3CH_2Cl = 5/1/1$. Le flux gazeux à la température réactionnelle et de 19l/h. A la sortie du réacteur, les gaz sont piégés et analysés par chromatographie en phase gazeuse. On observe la formation d'un produit dont le temps de rétention est identique à celui de la trifluoroéthylamine.

## Revendications

1. Composés chimiques caractérisé par le fait qu'ils répondent à la formule suivante sur sec :

$$(EO_4M), (Imp)p \quad (I)$$

avec E choisi dans le groupe des éléments de la colonne VB à l'exception de l'azote de préférence le phosphore,
- où M est un métal ou un mélange de métaux tel que :

$M = \alpha M_1^+ + \beta M_2^{++} + \gamma M_3^{3+} + \delta M_4^{4+}$ avec la relation $\alpha + 2z + 3\gamma + 4\delta = 3$

$M_1$ est choisi dans le groupe des éléments de la colonne I A et leurs mélanges, de préférence les métaux alcalins ;

$M_2$ est choisi parmi les éléments de transition divalents, les alcalino-terreux (Alt) et leurs mélanges ;

$M_3$ est choisi parmi les éléments de transition trivalents, les éléments de la colonne III B, les éléments à sous-couche électronique f et assimilés, et leurs mélanges ;

$M_4$ est choisi parmi les terres rares tetravalentes, le titane, le hafnium, l'étain, le germanium, et le silicium ;

17

$\alpha$ est un coefficient compris entre 0 et 3.

$\beta$ est un coefficient compris entre 0 et 3/2.

$\gamma$ est un coefficient compris entre 0 et 1, avantageusement au moins égal à 1/3.

delta est un coefficient compris entre 0 et 3/4, avantageusement entre 0 et 1/3 ;

Imp différent de ($EO_4M$) correspond à un composé d'imprégnation constitué de métalalcalino-terreux Alt, de préférence de métal alcalin Alc et leurs mélanges associés à un contreanion pour assurer la neutralité électrique ;

$\alpha + p$ étant en général inférieur ou égal à 3,3 et supérieur à $10^{-2}$, avantageusement compris entre 0,05 et 1, de préférence entre 0,05 et 1/2.

2.  Composés chimiques selon la revendication 1 caractérisé par le fait que le coefficient p est compris entre $10^{-2}$ et 1/3 avantageusement entre $1/2.10^{-1}$ et 1/4.

3.  Composés chimiques selon l'une des revendications 1 et 2 caractérisé par le fait que $M_3$ est choisi parmi les métaux à sous-couche électronique f et leurs mélanges et les mélanges les contenant, de préférence ledit métal $M_3$ est choisi parmi les terres rares (yttrium, lanthane, les lanthanides de préférence le lanthane), leurs mélanges et les mélanges les contenant.

4.  Composés chimiques selon l'une des revendications 1 à 3, caractérisé par le fait que il présente une structure monoclinique ou hexagonale de preference monoclinique d'origine hexagonale.

5.  Composés chimiques selon l'une des revendications 1 à 4 caractérisé par le fait que $Al_c$ représente le sodium, le potassium, le rubidium et le cesium et leurs mélanges.

6.  Composés chimiques selon la revendication 5 caractérisé par le fait que $Al_c$ est choisi dans le groupe du potassium, du cesium et de leurs mélanges.

7.  Catalyseurs de solvolyse d'un composé halogéné caractérisé par le fait qu'il est constitué au moins partiellement d'un composé répondant à la formule (I') suivante sur sec :

$$(EO_4M), (Imp)p \quad (I')$$

avec E choisi dans le groupe des éléments de la colonne VB à l'exception de l'azote de préférence le phosphore,

- où M est un métal ou un mélange de métaux tel que :

$M = \alpha M_1^+ + \beta M_2^{++} + \gamma M_3^{3+} + \delta M_4^{4+}$ avec la relation $\alpha + 2z + 3\gamma + 4\delta = 3$

avec $M_1$ est choisi dans le groupe des éléments de la colonne I A et leurs mélanges, de préférence les métaux alcalins ;

$M_2$ est choisi parmi les éléments de transition divalents, les alcalino-terreux (Alt) et leurs mélanges.

$M_3$ est choisi parmi les éléments de transition trivalents, les éléments de la colonne III B, les éléments à sous-couche électronique f et assimilés, et leurs mélanges.

$M_4$ est choisi parmi les terres rares tetravalentes, le titane, le hafnium, l'étain, le germanium, et le silicium.

$\alpha$ est un coefficient compris entre 0 et 3 avantageusement supérieur à 0,01.

$\beta$ est un coefficient compris entre 0 et 3/2.

$\gamma$ est un coefficient compris entre 0 et 1, avantageusement au moins égal à 1/3.

delta est un coefficient compris entre 0 et 3/4, avantageusement entre 0 et 1/3.

Imp correspond à un composé d'imprégnation basique constitué de métal alcalino-terreux Alt, de préférence de métal alcalin Alc et leurs mélanges associés à un contreanion pour assurer la neutralité électrique.

Le coefficient p est compris entre $10^{-2}$ et 1/3.

En outre, $\alpha + p$ est en général inférieur ou égal à 3,3 et supérieur à $10^{-2}$, avantageusement compris entre 0,05 et 1, de préférence entre 0,05 et 1/2.

8.  Catalyseurs selon la revendication 7 formé de corps catalytiques constituée partiellement de composé selon l'une des revendications 1 à 6 caractérisé par le fait que ledit composé se trouve majoritairement à la surface dudit corps catalytique.

9.  Catalyseurs selon la revendication 8, caractérisé par le fait que le reste dudit corps catalytique peut être réalisé en composés choisis dans le groupe constitué par les phosphates, les hydrogénophosphates et des composés de formule : $EO_4M$ (II)

10. Catalyseurs selon les revendications 7 à 9 caractérisé par le fait que les corps catalytiques sont entièrement réalisés en composé(s) selon l'une des revendications 1 à 6.

11. Procédé de fabrication de catalyseurs selon la revendication 10, caractérisé par le fait qu'il comporte les étapes suivantes :
   a) on réalise la synthèse d'un composé de formule (II) : $EO_4M$
   puis de préférence sans séparer $EO_4M$ du milieu réactionnel :
   b) on introduit l'imprégnant dans le milieu réactionnel ;
   c) on sépare l'éventuel liquide résiduel d'avec le solide réactionnel ;
   d) on sèche et calcine (température au moins égale à 100° C).

12. Procédé de synthèse des composés selon les revendications 1 à 6, selon une technique en elle-même connue caractérisé par le fait que ladite synthèse du composé est réalisée en présence d'un alcalin $Al_c$ et d'un contreanion assurant la neutralité électrique.

13. Procédé de traitement d'un halogénure d'alcoyle, caractérisé par le fait que l'on fait réagir ledit halogénure d'alcoyle, lequel alcoyle est de préférence électroattracteur, en présence d'un catalyseur sel on l'une des revendications 7 à 10.

14. Procédé selon la revendication 13, caractérisé par le fait que la réaction a lieu en phase gazeuse.

15. Procédé selon l'une des revendications 13 et 14, caractérisé par le fait que ledit halogénure d'alcoyle répond à la formule suivante :

$$R - CX_2 - CH_2Y \quad (III)$$

   – où les X sont choisis indépendamment parmi les halogènes de préférence parmi le fluor et le chlore;
   – où Y est un halogène de préférence le fluor, le chlore, le brome ou l'iode ;
   – où R est un alcoyle, de préférence électroattracteur, ou un halogène de préférence chlore ou fluor.

16. Procédé selon l'une des revendications 13 à 15, caractérisé par le fait que la température est comprise entre 200 et 800°C, de préférence entre 400° et 600°C.

17. Procédé selon l'une des revendications 13 à 16, caractérisé par le fait que le dit traitement est une solvolyse le rapport molaire entre solvants et substrats étant compris entre 1 et 100, de préférence entre 2 et 10.

18. Procédé selon la revendication 17, caractérisé par le fait que la pression est comprise entre 1 et 100 atmosphères ($10^5$ à $10^7$ pa) de préférence de 1 à 20.

19. Procédé selon l'une des revendications 15 à 18, caractérisé par le fait que le débit catalytique exprimé en gramme de substrats par gramme de catalyseurs, est compris entre 0,05 et 10.

20. Procédé selon l'une des revendications 15 à 19, caractérisé par le fait que la densité apparente du catalyseur est comprise entre 0,5 et 2.

21. Procédé selon l'une des revendications 15 à 20, caractérisé par le fait que R est égal à F.

22. Procédé selon l'une des revendications 15 à 21, caractérisé par le fait que les deux X de la formule (III) sont des atomes de fluor.

23. Procédé selon l'une des revendications 15 à 22, caractérisé par le fait que Y représente un chlore.

24. Procédé selon l'une des revendications 15 à 23, caractérisé par le fait que le solvant est choisi parmi les réactifs nucléophiles, polaires protiques.

25. Procédé selon la revendication 24, caractérisé par le fait que ledit solvant est choisi parmi les amines primaires ou secondaires et parmi les alcools y compris les phénols.

26. Procédé selon la revendication 23, caractérisé par le fait que ledit solvant est de l'eau.

27. Procédé selon l'une des revendications 13 à 16, caractérisé par le fait que le dit traitement est une deshydrohalogènation avantageusement à une pression inferieure à 10 atmosphères ($10^6$ Pa).

## FIG. 1a

O

Terre rare

P

## FIG. 1b

P

O

Terre rare

Alcalin

Office européen
des brevets

**RAPPORT DE RECHERCHE EUROPEENNE**

Numero de la demande

EP 91 40 0227

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 228 898 (NIPPON SHOKUBAI)<br>--- | | B 01 J 27/18<br>C 07 C 29/124<br>C 01 B 25/26 |
| A | FR-A-2 192 083 (CFR)<br>--- | | |
| A | US-A-4 560 798 (FORD et al.)<br>--- | | |
| A | CH-A- 474 452 (KNAPSACK)<br>--- | | |
| A | US-A-4 729 978 (SAWICKI)<br>----- | | |

**DOMAINES TECHNIQUES RECHERCHES (Int. Cl.5)**

B 01 J
C 07 C
C 01 B

Le présent rapport a été établi pour toutes les revendications

| Lieu de la recherche | Date d'achèvement de la recherche | Examinateur |
|---|---|---|
| LA HAYE | 12-04-1991 | LO CONTE C. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
........................................................................
& : membre de la même famille, document correspondant

EPO FORM 1503 03.82 (P0402)